(19) Europäisches Patentamt / European Patent Office / Office européen des brevets

(11) **EP 4 785 876 A1**

(12) **EUROPEAN PATENT APPLICATION**

(43) Date of publication:
   **05.08.2026 Bulletin 2026/32**

(21) Application number: **26155825.8**

(22) Date of filing: **02.02.2026**

(51) International Patent Classification (IPC):
   **A61B 8/08** (2006.01)    **A61B 8/12** (2006.01)
   **A61B 5/00** (2006.01)    **A61B 8/00** (2006.01)
   **G06T 7/00** (2017.01)

(52) Cooperative Patent Classification (CPC):
   **A61B 8/0841; A61B 5/0059; A61B 8/0883;
   A61B 8/12; A61B 8/4245; A61B 8/463; A61B 8/52;
   G06T 7/0012; G06T 7/70; G06T 7/73;** A61B 8/06;
   A61B 8/4488; A61B 8/483; A61B 8/5223

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **GE KH LA MA MD TN**

(30) Priority: **04.02.2025 US 202563753522 P
   17.06.2025 US 202519240516**

(71) Applicant: **Siemens Medical Solutions USA, Inc.
   Malvern, PA 19355 (US)**

(72) Inventors:
   • **KIM, Young-Ho
     Princeton, NJ, 08540-6632 (US)**
   • **HUH, Jaeyoung
     Princeton, NJ, 08540-6632 (US)**
   • **KAPOOR, Ankur
     Princeton, NJ, 08540-6632 (US)**

(74) Representative: **HKW Intellectual Property PartG mbB
   Theresienhöhe 12
   80339 München (DE)**

(54) **ULTRASOUND IMAGING CATHETER POSE ESTIMATION VIA ANATOMY**

(57)    For pose estimation of an imaging catheter, an image or other scan data from the imaging catheter is input to a machine-learned model, which is configured by training to output the pose of the imaging catheter. A position sensor is not needed as only an image as input is sufficient for accurate pose estimation. Manual adjustment or reliance on user expertise is limited as the machine-learned model directly provides the pose.

FIG. 1

EP 4 785 876 A1

**Description**

PRIORITY CLAIM

This application claims priority to U.S. provisional application serial number 63/753,522, filed February 4, 2025, which is incorporated by reference.

BACKGROUND

The present embodiments relate to imaging with a catheter, such as an intracardiac echocardiographic (ICE) catheter. ICE-based imaging is a widely used real-time, high-resolution imaging modality that provides critical visualization of cardiac structures from within the heart. ICE-based imaging plays a vital role in both Electrophysiology (EP) procedures and Structural Heart Disease (SHD) interventions, allowing clinicians to perform complex cardiac procedures with improved precision and safety.

In EP procedures, accurate catheter localization is crucial. CARTO (Biosense Webster Inc., USA) integrates electro-magnetic (EM)-based tracking with anatomical mapping to enable precise ICE catheter navigation. EM-based tracking relies on static anatomical maps and is susceptible to magnetic interference, causing position drift or inaccuracies. SHD interventions typically lack position-tracking systems, requiring operators to manually adjust ICE imaging to explore key anatomical structures such as the left atrial appendage (LAA), pulmonary veins (PV), and atrial septum. This user-adjustment process relies heavily on operator experience and may require frequent adjustments, increasing procedural complexity.

Recent advancements in artificial intelligence (AI)-driven ICE catheter navigation have introduced autonomous view recovery to reduce operator workload and improve procedural efficiency. Automated view recovery systems allow clinicians to bookmark critical imaging views and return to them at the push of a button, facilitating efficient and repeatable ICE imaging during interventions. This AI-based systems aims to enhance ICE manipulation efficiency, particularly for less experienced operators, but does not provide catheter localization.

SUMMARY

By way of introduction, the preferred embodiments described below include methods, systems, non-transitory computer readable media, and improvements for pose estimation of an imaging catheter. An image or other scan data from the imaging catheter is input to a machine-learned model, which is configured by training to output the pose of the imaging catheter. A position sensor is not needed as only an image as input is sufficient for accurate pose estimation. Manual adjustment or reliance on user expertise is limited as the machine-learned model directly provides the pose.

In a first aspect, a method is provided for pose estimation of an ultrasound imaging catheter. An ultrasound system images an internal region of a patient with the ultrasound imaging catheter. The imaging provides an image of the internal region. A machine-learned model estimates a pose of the ultrasound imaging catheter. The pose is estimated by the machine-learned model in response to input of the image to the machine-learned model. An indication of the pose is displayed.

In a second aspect, an ultrasound system is provided for pose estimation. An intracardiac echocardiogram (ICE) catheter with a transducer is configured for ultrasound scanning from within a cardiac system of a patient. An image processor is configured to determine a position and orientation of the ICE catheter by input of a scan from the ICE catheter to a neural network. The neural network is configured by training to output the position and orientation in response to input of the scan. A display is configured to display the position and orientation.

In a third aspect, a system is provided for pose estimation of an imaging catheter. An image processor is configured to generate a pose of the imaging catheter. The pose is generated by a neural network configured to output the pose in response to input of an image from the imaging catheter to the neural network. A display is configured to display the pose.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, computer program, or non-transitory computer readable storage medium.

For example, the imaging processor of the second and third aspect may be implemented to carry out the step of estimating a pose according to the first aspect. A pose may be or include a position and an orientation; a scan of an ICE catheter may be considered an image; a machine-learned model may be or include a neural network.

The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 a flow chart diagram of one embodiment of a method for estimation of a pose of an imaging catheter;

Figure 2 illustrates an example machine-learned model for input of an image and output of position and orientation as the pose;

Figure 3 illustrates a graphic indication of pose relative to anatomy;

Figure 4 is a block diagram of one embodiment of a system for pose estimation; and

Figure 5 illustrates an example neural network.

DETAILED DESCRIPTION OF THE DRAWINGS AND PRESENTLY PREFERRED EMBODIMENTS

The pose (e.g., position and orientation) of an ICE or another imaging catheter is accurately estimated using an image (e.g., ICE image). Precise localization is provided for EP, SHD, or other interventions or procedures. Since the image is used, anatomy-aware pose estimation is provided. The pose may be estimated using only an image, eliminating the need for external sensors. The catheter position is expressed in relation to a key anatomical landmark rather than a pre-defined map. This method enhances navigation intuition, making it easier to reach target anatomical structures. It is particularly beneficial for position-tracking-free procedures and may complement existing mapping systems like CARTO, providing real-time anatomical understanding for improved localization.

In one ICE implementation, the AI-based pose estimation directly derives the positions and orientations of an ICE catheter from ICE images, eliminating the need for external tracking sensors. ICE images, precise position data, and anatomical structures are integrated through the AI for pose estimation. The pose is directly output rather than output of a view label. The pose may be for the full 6 degrees of freedom (DOF) for position and orientation without relying on external tracking. Various machine-learned models may be used, such as a neural network or vision transformer, for the ICE catheter pose estimation.

Unlike CARTO, which relies on EM tracking, only an ICE image is needed for pose estimation. No reliance on EM sensors reduces magnetic interference and drift issues, providing independence from external tracking systems. Unlike manual ICE manipulation, which depends on operator expertise, the system provides real-time, AI-driven localization. Improved procedural efficiency is provided by reduction in manual ICE adjustments. The pose estimation directly from the AI may be used alone or complementary to existing tracking (e.g., EM-based tracking). Unlike prior AI-based navigation, which assist in view guidance, the pose is directly estimated for real-time anatomical localization.

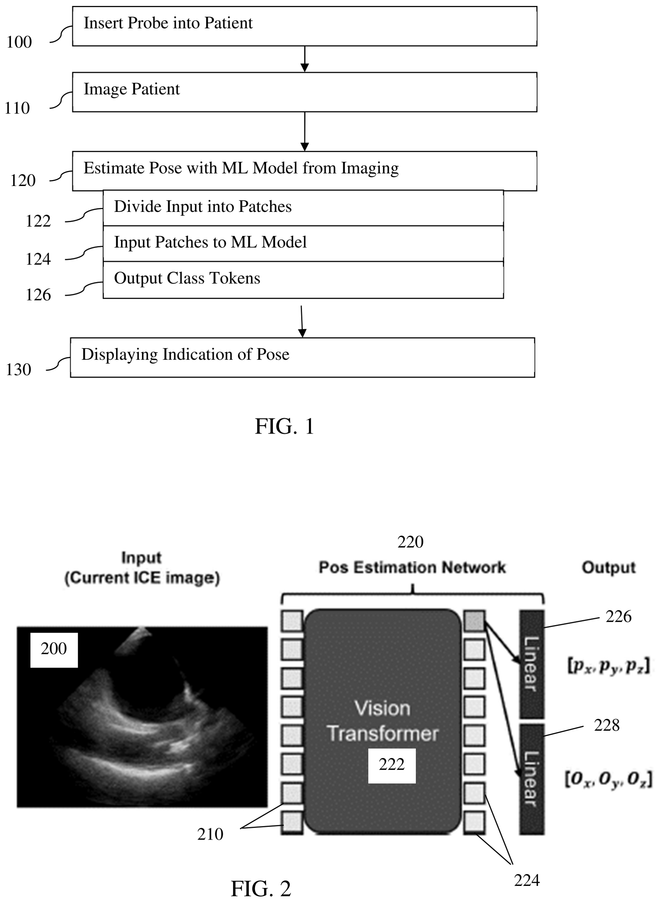

Figure 1 is a flow chart diagram of one implementation of a method for pose estimation of an imaging catheter, such as an ultrasound imaging catheter (e.g., ICE catheter). AI directly estimates the pose from input of an image from the imaging catheter. Real-time, accurate pose estimation limits manual adjustments, increasing procedural efficiency without the need for an extra sensor.

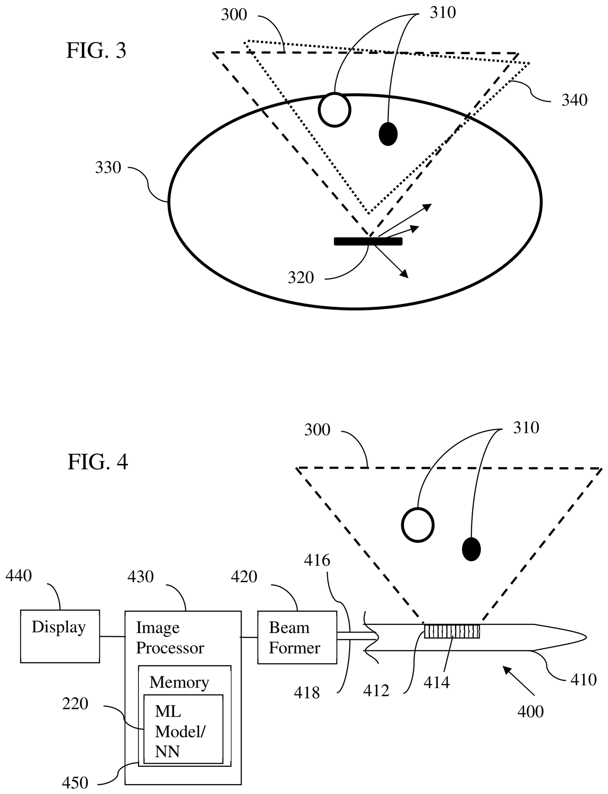

The system of Figure 4 or another system implements the acts of Figure 1. An image processor (e.g., computer, workstation, server, or imaging system) may be used. An imaging system, such as an ultrasound system, may be used. The physician performs act 100, and then the imaging (e.g., ultrasound) system performs acts 110 and 130. A computer, workstation, server, or another image processor may perform acts 120-126.

Additional, different, or fewer acts may be provided. For example, acts for configuring the ultrasound imaging system and/or acts for diagnosis or treatment are included. As another example, act 100 is not provided, such as where the imaging catheter is already in the patient or where the method is directed to the actions or process of the ultrasound scanner. In yet another example, acts 122, 124, and/or 124 are not provided, such as where other acts are used for AI-based estimation of the pose.

The acts are performed in the order shown (top to bottom or numerically) or a different order.

The method may be performed using any imaging catheter, such as a catheter using optical or infrared imaging. In one implementation, the method is performed using ultrasound imaging, such as with an ICE or other catheter having an ultrasound transducer. The ICE imaging example is used below.

In act 100, the catheter probe is inserted into a patient. The probe is inserted into a lumen, such as a blood vessel. For example, the probe is an ICE catheter inserted into the cardiac system to navigate to the heart. The tip of the probe is positioned for imaging tissue of interest. In one implementation, a transseptal puncture is performed, and an ICE imaging probe is deployed to the target position. Guide wires, translation, and/or rotation are used to steer the probe to a position for imaging. The transducer, imaging array, or imaging sensor of the probe is positioned so that a scan plane or volume includes the tissue of interest for visualization during EP, SHD, or another intervention.

In act 110, the ultrasound system images the tissue to be treated or diagnosed and any ablation or treatment instrument (e.g., ablation catheter). An imaging array of the ICE catheter images an internal region of the patient from within the patient. The ultrasound scanner images in the plane defined by the imaging array (transducer). Volume imaging may be provided using a multidimensional array, shaped 1D array, or movement of the 1D array.

The array connects with the beamformer to scan the patient. The scan region is scanned with ultrasound, and an ultrasound image or images of tissue and/or fluid of the patient is generated in act 110. Any imaging (e.g., B-mode and/or flow or color mode) may be used. The imaging generates one or more images, such as images representing tissue and any other objects (e.g., ablation catheter) in a two or three-dimensional region.

The images may be image frames of scan data prior to scan conversion (e.g., beamformed data prior to or after detection) or after scan conversion. The image may be formatted for display or a spatial representation not yet formatted for display (e.g., not scan converted and/or not color or grayscale mapped).

In act 120, an image processor, such as a processor of the ultrasound scanner, estimates a pose of the imaging catheter (e.g., ICE catheter). The pose is estimated as the position and/or orientation of the imaging catheter or another component of the imaging catheter. For example, the pose of the ICE catheter is estimated as a position and orientation of a transducer or tip region of the ICE catheter. The pose is estimated in any number of degrees of freedom (DOF), such as six DOF (three for position and three for orientation).

The pose is estimated relative to anatomy of the patient. For example, the pose is estimated relative to a left atrium (LA), such as a center of the LA. Another anatomical reference may be used, such as a spetum, ostium, valve, heart chamber, or vein. By estimating relative to anatomy of the patient, consistent and interpretable localization is provided. In other implementations, the pose is estimated relative to another coordinate system, such as an EM sensor system.

The image processor implements a machine-learned model to estimate the pose. The image processor instantiates or executes the machine-learned model, inputs to the model, and generates the output using the model. The machine-learned model, as implemented by the image processor, estimates the pose in response to input of the image to the machine-learned model.

The machine-learned model may be any now known or later developed machine-trained model, such as a Bayesian network, neural network, or a support vector machine. In one implementation, the machine-learned model is a neural network trained with deep learning.

The arrangement or architecture of the machine-learned model dictates the input and output. For pose estimation, the input is the image or a sequence of images. Other inputs may be provided, such as patient information, breathing sensor signal, and/or electroencephalogram (EKG) signal. The output is the pose. The machine-learned model directly outputs the pose of the imaging catheter in response to the input.

In one implementation, the machine-learned model and the image processor estimate the pose free of input from a position sensor. An EM or other position sensor for the imaging catheter is not used. The machine-learned model estimates the pose in response to input of only the image or images. Real-time pose estimation is provided by repetitively inputting images as the images are formed or acquired. The pose for the imaging catheter for each image is output. The model architecture is arranged to receive the original image or sequence of images (image or scan data) and output the pose.

The machine-learned network is a fully connected, convolutional, or another neural network. Any network structure may be used. Any number of layers, nodes within layers, types of nodes (activations), types of layers, interconnections, learnable parameters, and/or other network architectures may be used.

In one approach, the neural network is configured as a vision transformer (ViT). Any vision transformer architecture to receive an image as input and output a classification, such as pose, may be used. An attention mechanism is provided using tokens, where each layer contextualizes the token. The transformer is an encoder only arrangement (e.g., BERT-like encoder-only) for processing tokens, but other transformer architectures (e.g., encoder-decoder) may be used. A masked autoencoder, self-distillation with no labels (DINO), shifted windows (Swin), TimeSformer, ViT-VQGAN, CoAtNet, CvT, data-efficient ViT (DeiT), or another vision transformer may be used. Single matrix multiplication may be used. A masked autoencoder may be used. A convolutional neural network or combination of convolutional neural network and ViT may be used, such as using the convolutional neural network as a preprocessor to the ViT neural network.

Any or no token mechanism may be used, such as class token (CLS). In another implementation, global average pooling (GAP) is used. Multihead attention pooling may be used. Other ViT approaches and corresponding architectures may be used.

Figure 2 shows one example neural network 220 for estimating the pose. The input is a single or multiple images 200, such as one ultrasound image generated for ICE imaging. The image 200 is a scan converted display image, but other ultrasound-based spatial representations (e.g., detected values in a scan format) may be used.

The image 200 is divided into patches 210. For example, the image processor divides the input image 200 in act 122 into 16x16 patches 210 with spatial encoding representing the spatial relationship of each patch 210 to the other patches 210. Each ICE image 200 is patchified into 16×16 blocks and embedded into a 768- or other dimensional space with the positional encoding. Other size patches may be used.

The patches 210 and spatial (position) encoding are input to the ViT 222 in act 124. In response to the input, the ViT 222 generates the output class (e.g., [CLS]) tokens 224 as output in act 126. The class tokens 224 are appended and processed through the ViT 222, allowing the model to capture complex spatial relationships between the ICE image 200 and anatomical landmarks. One token 224 is provided for each patch 210. Other token arrangements may be used. The pose is generated in act 120 from the class tokens 224.

In one implementation shown in Figure 2, separate outputs (e.g., multi-task classification) are provided, one for the position and one for orientation. One linear layer or layers 226 generate the position from the tokens 224, and another linear layer or layers 228 generate the orientation from the same tokens 224. In other implementations, one layer or layers outputs both position and orientation. Pose may be just position, just orientation, or position and orientation. Less than six DOF may be estimated, such as where the imaging catheter is held or fixed in one or more DOF or other estimation is provided for the other DOF. Another pose parameterization may be used.

The ViT-based neural network enables global feature extraction from the image 200. The machine-learned model or neural network 220 provides direct pose estimation. The multi-output structure (e.g., separate linear layers 226, 228) directly generates the pose estimation. Unlike conventional methods that predict only image-to-view correspondences, this model directly outputs pose (e.g., position + orientation). The class token 224 outputs are passed through two separate linear layers: one branch (layer 226) predicts position (P^\hat{P}P^), and the other branch (layer 228) predicts orientation (O^\hat{O}O^). This multi-output approach allows the system to efficiently infer both spatial location and directional information for ICE catheter navigation.

Machine training uses the defined architecture, training data, and optimization to learn values of learnable parameters of the architecture based on the samples and ground truth of training data. For training the model to be applied as a machine-learned model, training data is acquired and stored in a database or memory. The training data is acquired by expert review, aggregation, mining, loading from a publicly or privately formed collection, transfer, and/or access, such as collecting from patient medical records. Ten, hundreds, or thousands of samples of training data are acquired. The samples are from scans of different patients and/or phantoms. Simulation may be used to form the training data. The training data includes many samples of the desired output (ground truth), such as pose, and the input, such as ICE images. In one implementation, a large dataset of ICE images, each annotated with a pose, is used to train.

A machine (e.g., image processor, server, workstation, or computer) machine trains the neural network or another machine learning model to estimate the pose. The training uses the training data to learn values for the learnable parameters (e.g., convolution kernels, node weights, link weights, and/or settings of activation functions) of the network. The training determines the values of the learnable parameters of the network that most consistently output close to or at the ground truth given the input samples. In training, the loss function may be a mean squared error loss between predicted poses and ground truth poses. Other loss functions, such as L1, cross entropy, or L2, may be used. Adam, gradient descent, another first order optimization algorithm, or another function is used for optimization (e.g., to minimize the loss or maximize a gain).

In one implementation to estimate ICE catheter pose purely from ICE images, the ViT (deep learning model) is trained to learn the spatial relationships between ICE catheter pose and their corresponding anatomical view images. A dataset is collected from a well-established clinical environment and processed using the CARTO mapping system for the ground truth pose. The dataset includes ICE images paired with their corresponding position and orientation information, as well as cardiac mesh data. For example, the mesh is of the left atrium (LA) surface. Any number of samples may be acquired, such as multiple images from different poses in hundreds or thousands of patients. The dataset is split into training, validation, and testing sets (e.g., 851 subjects, with the dataset split into 793 for training, 25 for validation, and 33 for testing). Where each dataset is collected independently, each may have their own world coordinate system. To ensure consistency across samples, all position and orientation data are normalized relative to a same anatomical reference (e.g., the center of each left atrium (LA) mesh). This normalization allows the neural network to be trained to estimate pose relative to anatomy of the patient. Other anatomy may be used. The transformation from the world coordinate-based transducer state ($S_{\mathrm{w}}^{tr}$) to the center of the LA mesh-based state ($S_{\mathrm{m}}^{tr}$) is achieved using a transformation matrix ($T_{w}^{m}$). This normalization process may be expressed as follows:

$$S_{\mathrm{m}}^{tr} = (T_{w}^{m})^{-1} * S_{\mathrm{w}}^{tr}.$$

The dataset includes any number of training samples, such as 18,305 training samples with 813 samples for validation and 823 for testing.

In this implementation, the ViT-based neural network as shown in Figure 2 is trained to capture global spatial relationships within ICE images. Each ICE image is divided in 16 × 16 patches and embedded into 768 dimensional vectors with positional encoding. A class token (*[CLS]*) is appended, and the sequence is processed through the transformer network. The *[CLS]* token output is passed through two separate linear layers to predict position P and orientation $\hat{O}$ as $\hat{P}$, $\hat{O}$=N(I).

The model is trained as a multi-task model using Mean Squared Error (MSE) loss, with a total loss function:

$$l_{total} = l_{mse}(\hat{P}, P) + \lambda * l_{mse}(\hat{O}, O),$$

where $\lambda$ = 2 or another value balances position and orientation errors. Other loss functions and/or combinations of losses may be used. Only one output may be provided, so that the loss function is for the output rather than multiple tasks (e.g., position and orientation). The network 220 is trained for any number of epochs, such as 140 epochs, with any batch size, such as a batch size of 16 The training may be implemented in PyTorch or another machine learning platform and be conducted on an NVIDIA A100 GPU, another GPU, or another machine training machine (processor).

Once trained, the machine-learned model or trained neural network is stored for later application. The training determines the values of the learnable parameters of the network. The network architecture, values of non-learnable parameters, and values of the learnable parameters are stored as the machine-learned network. Copies may be distributed, such as to ultrasound scanners, for application. Once stored, the machine-learned network may be fixed. The same machine-learned network may be applied to different patients, different scanners, and/or different treatments.

The machine-learned network may be updated. As additional training data is acquired, such as through application of the network for patients and corrections by experts to that output, the additional training data may be used to re-train or update the training.

By training using a combination of the image (input sample), precise pose (e.g., position and orientation ground truth for each sample), as well as anatomical reference information (e.g., training relative to an anatomical reference such as the LA mesh for the ground truth), the trained network uses images to directly and accurately determine poses relative to anatomy. Unlike prior approaches that rely solely on pre-registered anatomical maps or external EM-tracked data, the integration of ICE images with real-time anatomical understanding provided by the training or trained network results in pose normalized relative to anatomy, ensuring consistent and interpretable localization.

In act 130, the image processor generates an image with an indication of the pose, and a display screen displays the indication of the pose as a cue to the viewer. The pose relative to the anatomy is displayed. The indication of the pose is generated from the imaging of act 110 based on the image processing used to generate the pose in act 120.

The indication is a graphic, highlighting, enhancement, annotation, or alphanumeric text for the angle and/or location of the shaft or sensor of the imaging catheter relative to an anatomical reference (e.g., center of LA). Figure 3 shows an example. The scan plane 300 images anatomy 310. The transducer 320 is part of the imaging catheter. The pose of the transducer 320 relative to anatomical structure is displayed. In this example, the indication of pose is of arrows or vectors from the transducer 320 relative to a heart chamber 330. In one approach, the graphics for both position and orientation are rendered in a three-dimensional coordinate system anchored to a consistent anatomical reference, such as the center of the left atrium (LA)(e.g., rendered LA mesh -anatomy 330 rendered as a three-dimensional mesh instead of a two-dimensional cross-section). This ensures that pose indications are interpretable and comparable across patients and over time. The position of the catheter or transducer 320 is shown as a point or vector in this anatomy-relative space. The orientation is shown via vector direction or angular deviation relative to the reference axes, representing the heading of the imaging plane 300. For example, the home view (default ICE imaging direction) may serve as the reference orientation, with pose vectors indicating deviation from that canonical alignment. This anatomical coordinate system enables consistent visualization of the catheter's 6-DOF pose and supports accurate procedural navigation. In another approach, the magnitude and direction of the arrows indicates the pose. Other graphics may be used, such as a connecting line showing a shortest path from the transducer 320 to the anatomical reference (e.g., center of chamber 330) with color, width variation, arrows, or other indication of angle or orientation relative to a reference orientation of the chamber 330. Any graphic showing the pose of the imaging catheter relative to any anatomy of the patient may be used.

As another example, the angle and distance between the imaging catheter and anatomy (e.g., vein or ostium) is displayed. These and/or other measurements assessing the quality of imaging catheter placement relative to anatomy are displayed. Both the angle and distance between the anatomy and the catheter are used for proper imaging catheter placement.

In another example, text or another graphic overly indicates the position and orientation in a 3D coordinate system. The 3D coordinate system has an origin at the anatomical reference.

The viewer plans for a given position and orientation relative to the anatomical reference. By indicating the pose, the viewer may confirm proper positioning of the imaging catheter for monitoring operation and/or treatment of the anatomy. By providing real-time feedback on imaging catheter pose using the machine-learned model estimation, the operator is aided in establishing and maintaining a desired view plane relative to anatomy.

Acts 110-130 are repeated. The pose may be estimated for each image generated. Less frequent pose indication may be used, such as every other image, every third image, or less frequent. The display of pose in act 130 may be updated for each repetition.

In an example implementation, the method is validated through qualitative and quantitative evaluations using the paired dataset. In a qualitative assessment, Figure 3 illustrates the alignment between the predicted imaging scan region 300 and the target scan region 340 within a two-dimensional anatomical representation. The assessment may be done in three dimensions but is shown in two dimensions for simplicity. In a three-dimensional assessment using the LA mesh as the anatomical reference, the model accurately visualizes key structures, such as the left atrial appendage (LAA) and right pulmonary vein (RPV), with minimal positional and orientational errors.

In a quantitative evaluation, Table I summarizes the errors, with an average positional error of 9.48 mm and orientation errors of (16.13, 8.98, 10.47) degrees across the x-, y-, and z-axes. These results confirm the high accuracy of pose estimation using the machine-learned model with input of an image and output of pose in estimating catheter position and orientation.

TABLE I Prediction error

|  | Position | Orientation |
| --- | --- | --- |
| Mean error | 9.48 [mm] | (16.13, 8.98, 10.47) [degree] |
| Std | 5.96 [mm] | (42.31, 9.47, 14.81) [degree] |

Other errors may result, depending on the training data, model architecture, training process, and/or anatomical reference.

Figure 4 shows a system for pose estimation of an imaging catheter. Any type of imaging catheter and corresponding computer or system to image and estimate pose from the images may be used. In the example of Figure 4, the system is an ultrasound system for pose estimation of an ICE or another ultrasound catheter. The ultrasound system is used for imaging from the transducer 412 of the ICE catheter 400 as well as estimating pose of the ICE catheter 400 (e.g., transducer 412) relative to anatomy. The ICE catheter 400 generates images, from which relative position of the ICE catheter 400 to the anatomy is determined for indicating the pose to the viewer. Other types of imaging catheters and corresponding imaging systems may be used.

The ultrasound imaging system includes the ICE catheter 400 (e.g., array 412 of elements 414 and a housing 410) and an ultrasound scanner (e.g., a beamformer 420, an image processor 430, and a display 440). Additional, different, or fewer components may be provided. For example, the system includes the image processor 430 and display 440 without the beamformer 420 and/or ICE catheter 400, such as where the image processor 430 operates on images formed by another device. The transducer array 412 and ICE catheter 400 releasably connect with the ultrasound scanner or imaging system.

The ICE catheter 400 includes the housing 410, the array 412 of elements 414, the conductors 416, and one or more guide wires 418. Additional, different, or fewer components may be provided. For example, a port or tube for inserting and/or withdrawing fluid from the housing 410 is included. As another example, one or more markers (fiducials) for position determination are included.

The housing 410 is a sleeve of plastic or other material for insertion into a patient. For example, the housing 410 is formed from Pebax. Other materials, such as other Nylons or biologically neutral (or biocompatible) materials, may be used. The housing 410 is sealed over the array 412 to separate fluids of the patient from the interior of the catheter 400.

The imaging array 412 is in or on the ICE catheter 400. The array 412 is a transducer configured for ultrasound scanning from within a cardiac system of a patient. The array 412 has a plurality of elements 414, electrodes, and a matching layer. Additional, different, or fewer components may be provided, such as a backing block. For example, two or more matching layers are used. As another example, a semiconductor chip (e.g., application specific integrated circuit) is stacked with the array 412 in the catheter housing 410.

The elements 414 may contain piezoelectric material. Alternatively, a microelectromechanical device, such as a flexible membrane, is used. Any now known or later developed ultrasound transducer may be used.

In one embodiment, the array 412 is a 1D array. The elements 414 are distributed along a straight or curved line to form the 1D array of elements 414. In other embodiments, the array 412 is a 1.5D or 2D array (multidimensional). In yet other embodiments, any array having one dimension greater than the width (diameter) of the probe body and another dimension less than the width may be used. For example, a planar imaging array produced as a capacitive micromachined ultrasound transducer (CMUT) where each element is composed of a matrix of micro-elements is used. A helical array, 2D array, or 1D array rotated to different positions may be used for 3D imaging. The 1D array in one position may be used for 2D imaging.

The array 412 is positioned distally from the steering section of the housing 410 of the probe 100. The array 412 is in or near a tip of the catheter housing 410. Other positions may be provided. The array 412 is positioned along the longitudinal axis within the housing 410.

The ultrasound scanner (e.g., beamformer 420, image processor 430, and/or display 440) is configured for ultrasound imaging. The array 412 is used to form an aperture for the scan plane 300. The beamformer 420 uses elements 414 of the array 412 to scan an image plane or volume. Conductors 416 connect the array 412 to the beamformer 420 for imaging. The beamformer 420 includes a plurality of channels for generating transmit waveforms and/or receiving signals.

The image processor 430 is a detector, filter, processor, application specific integrated circuit, field programmable gate array, digital signal processor, control processor, controller, scan converter, three-dimensional image processor, graphics processing unit, AI processor, tensor processor, analog circuit, digital circuit, or combinations thereof. The image processor 430 receives beamformed data and generates images on the display 440. In one implementation, the image processor 430 is one device for imaging and estimating pose. In another implementation, the image processor 430 is a combination of multiple devices that generate the ultrasound image (e.g., detector and scan converter) and estimate pose from the ultrasound scanning (from scan data and/or other images) (e.g., general processor).

The image processor 430 is configured by firmware, software, and/or hardware. The image processor 430 is configured to generate a pose of the imaging catheter 400. The pose is generated by a neural network 220 configured to output the pose in response to input of an image from the imaging catheter 400 to the neural network 220. The neural network 220 is configured, at least in part, by previous training and architecture, to generate the pose in response to input. The learned parameters of the neural network 220 are applied to the input and features derived therefrom to generate the output.

In one implementation, the image processor 430 is configured to determine a position and orientation of the ICE catheter as the pose. The position and orientation are determined by input of a scan from the ICE catheter 400 to the neural network 220. The neural network 220 is configured to output the position and orientation in response to input of the scan. The scan is a spatial representation of the patient, such as beamformed data prior to detection, detected data prior to scan conversion, scan converted data prior to color or grayscale mapping, a display image prior to display, a display image after display, or any other image in the ultrasound processing from beamformation to the display.

The pose is estimated for any part of the imaging catheter 400. For example, the pose of the sensor (e.g., transducer 412) is estimated. Alternatively, the pose of the tip or another part is determined.

The image processor 430 estimates the pose relative to anatomy. Based on the training, the neural network 220 outputs the pose relative to specific anatomy. The ground truth poses used in training are relative to specific anatomy, so the neural network in use outputs pose relative to that specific anatomy of the patient. For example, the LA center is used with the LA in a specific orientation relative to the patient. Other anatomical references may be used, such as an ostium, a valve, a vein, septum, or chamber.

The image processor 430 is configured to estimate the pose in response to the input of the scan free of input of or derived from a position sensor. The neural network 220 is configured to directly output pose in response to the input of only the image or only information without position information from a position sensor. The EM or other position sensor is not needed but may be used in combination with image-based estimation in other implementations. In alternative embodiments, the neural network 220 is configured to receive position information from a position sensor as well as the image to output the pose.

The neural network 220 may have any of various architectures defined to receive the specific input (e.g., scan data) to generate the specific output (e.g., position and orientation). In one approach, the neural network 220 is arranged as a vision transformer. For example, the vision transformer is configured to operate on patches of the spatial representation (scan or another image) with separate linear output layers configured to output the position and orientation, respectively, as the pose and in response to class tokens for the patches.

The instructions for implementing the processes, methods, and/or techniques discussed herein are provided on non-transitory computer-readable storage media or memories, such as a cache, buffer, RAM, removable media, hard drive, or other computer readable storage media (e.g., memory 450 of the ultrasound scanner). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU or system. Because some of the constituent system components and method steps depicted in the accompanying figures may be implemented in software, the actual connections between the system components (or the process steps) may differ depending upon the manner in which the present embodiments are programmed.

The display 440 is a monitor, liquid crystal display, television, tablet, mobile device, or another screen configured to display one or more images. Other displays, such as a printer, may be used. The display 440 is configured by loading an image into memory (e.g., display plane or buffer) to display the image. The display 440 is configured to display the pose, such as an image with an indication of pose. The display 440 is configured to display the position and orientation in one implementation, such as a graphic showing the position and orientation relative to anatomy of the patient. For example, a sequence of ultrasound images is displayed. Each image indicates the position and orientation of the transducer 412 relative to a center of the LA of the heart of the patient. The position and orientation are shown with a graphic and/or text. Other indication of pose may be used.

The user, upon viewing the pose, may steer the imaging catheter 400. The pose assists or helps with proper placement of the imaging catheter relative to the tissue. The resulting imaging more likely captures the desired objects or anatomy for diagnosis or treatment. By providing the pose continuously, regularly, or periodically, the continued placement of the imaging catheter relative to the anatomy is provided over time.

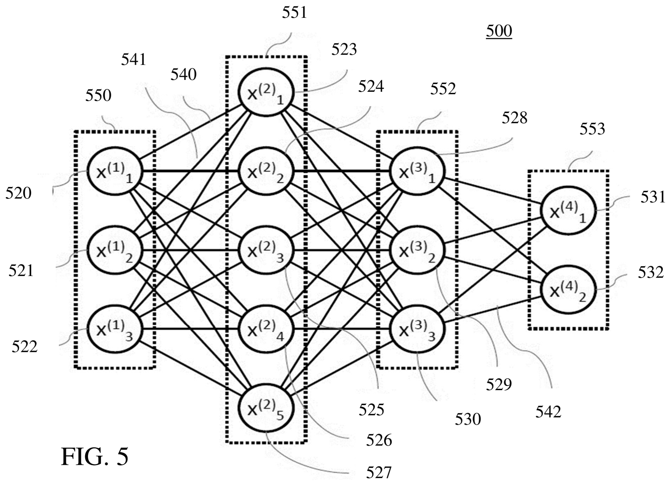

Figure 5 shows an embodiment of an artificial neural network 500, in accordance with one or more embodiments. Alternative terms for "artificial neural network" are "neural network," "artificial neural net," or "neural net." The artificial neural network 500 may be used in part in, for example, the one or more machine learning based networks utilized for compounding or generating 3D deformation. The artificial neural network 500 may be arranged or designed as a neural field for pose estimation.

The artificial neural network 500 includes nodes 520-532 and edges 540-542, wherein each edge 540-542 is a directed connection from a first node 520-532 to a second node 520-532. In general, the first node 520-532 and the second node 520-532 are different nodes 520-532, it is also possible that the first node 520-532 and the second node 520-532 are identical. For example, in Figure 5, the edge 540 is a directed connection from the node 520 to the node 523, and the edge 541 is a directed connection from the node 521 to the node 523. An edge 540-542 from a first node 520-532 to a second node 520-532 is also denoted as "ingoing edge" for the second node 520-532 and as "outgoing edge" for the first node 520-532.

In this embodiment, the nodes 520-532 of the artificial neural network 500 may be arranged in layers 550-553, wherein the layers may include an intrinsic order introduced by the edges 540-542 between the nodes 520-532. In particular, edges 540-542 may exist only between neighboring layers of nodes. In the embodiment shown in Figure 5, there is an input layer 550 including only nodes 520-522 without an incoming edge, an output layer 553 including only nodes 531 and 532 without outgoing edges, and hidden layers 551, 552 in-between the input layer 550 and the output layer 553. In general, the number of hidden layers 551, 552 may be chosen arbitrarily. The number of nodes 520-522 within the input layer 550 usually relates to the number of input values of the neural network 500, and the number of nodes 531-532 within the output layer 553 usually relates to the number of output values of the neural network 500. For a neural field, the number of layers and nodes corresponds to the coordinate system. Each node may be connected to adjacent nodes in any direction in a bidirectional edge. The input may be to all the nodes with output to all the nodes.

In one approach, the network architecture is a ViT with an encoder-only structure defined to receive image patches, process class tokens, and provide the class tokens to separate linear layers for output of position and orientation. Figure 5 shows the neural network 500 in a generalized form applicable to ViT. For a ViT, the arrangement of layers and/or nodes may be different. Any now known or later developed ViT-based neural network arrangement may be used.

A (real) number may be assigned as a value to every node 520-532 of the neural network 500. Here, $x^{(n)}_i$ denotes the value of the i-th node 520-532 of the n-th layer 550-553. The values of the nodes 520-522 of the input layer 550 are equivalent to the input values of the neural network 500, the value of the nodes 531-532 of the output layer 553 is equivalent to the output value of the neural network 500. Furthermore, each edge 540-542 may include a weight being a real number, in particular, the weight is a real number within the interval [-1, 1] or within the interval [0, 1]. Here, $w^{(m,n)}_{i,j}$ denotes the weight of the edge between the i-th node 520-532 of the m-th layer 550-553 and the j-th node 520-532 of the n-th layer 550-553. Furthermore, the abbreviation $w^{(n)}_{i,j}$ is defined for the weight $w^{(n,n+1)}_{i,j}$.

In particular, to calculate the output values of the neural network 500, the input values are propagated through the neural network. In particular, the values of the nodes 520-532 of the (n+1)-th layer 550-553 may be calculated based on the values of the nodes 520-532 of the n-th layer 550-553 by:

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise or through adjacent nodes through the neural network 500, wherein values of the input layer 550 are given by the input of the neural network 500, wherein values of the first hidden layer 551 may be calculated based on the values of the input layer 550 of the neural network 500, wherein values of the second hidden layer 552 may be calculated based in the values of the first hidden layer 551, etc.

To set the values $w^{(m,n)}_{i,j}$ for the edges, the neural network 500 has to be trained using training data. In particular, training data includes training input data and training output data (denoted as $t_i$). For a training step, the neural network 500 is applied to the training input data to generate calculated output data. In particular, the training data and the calculated output data include a number of values, said number being equal with the number of nodes of the output layer.

In particular, a comparison between the calculated output data and the training data is used to recursively adapt the weights within the neural network 500 (backpropagation algorithm). In particular, the weights are changed according to:

$$w'^{(n)}_{i,j} = w^{(n)}_{i,j} - \gamma \cdot \delta^{(n)}_j \cdot x^{(n)}_i$$

wherein y is a learning rate, and the numbers $\delta^{(n)}_j$ may be recursively calculated as:

$$\delta^{(n)}_j = \left( \sum_k \delta^{(n+1)}_k \cdot w^{(n+1)}_{j,k} \right) \cdot f' \left( \sum_i x^{(n)}_i \cdot w^{(n)}_{i,j} \right)$$

based on $\delta^{(n+1)}_j$, if the (n+1)-th layer is not the output layer, and

$$\delta^{(n)}_j = \left( x^{(n+1)}_k \cdot t^{(n+1)}_j \right) \cdot f' \left( \sum_i x^{(n)}_i \cdot w^{(n)}_{i,j} \right)$$

if the (n+1)-th layer is the output layer 553, wherein f' is the first derivative of the activation function, and $y^{(n+1)}_j$ is the comparison training value for the j-th node of the output layer 553.

Any vision transformer architecture to receive an image as input and output a classification, such as pose, may be used. An attention mechanism is provided using tokens, where each layer contextualizes the token. The transformer is an encoder only arrangement (e.g., BERT-like encoder-only) for processing tokens, but other transformer architectures (e.g., encoder-decoder) may be used. A masked autoencoder, self-distillation with no labels (DINO), shifted windows (Swin), TimeSformer, ViT-VQGAN, CoAtNet, CvT, data-efficient ViT (DeiT), or another vision transformer may be used. Single matrix multiplication may be used. A masked autoencoder may be used. A convolutional neural network or combination of convolutional neural network and ViT may be used, such as using the convolutional neural network as a preprocessor to the ViT neural network. Any or no token mechanism may be used, such as class token [CLS]. In another implementation, a global average pooling (GAP) is used. Multihead attention pooling may be used. Other ViT approaches and corresponding architectures may be used.

Listed below are various Illustrative Embodiments. The Illustrative Embodiments summarize different combinations of aspects or features. Other combinations of any of the aspects or features with any other one or more of the aspects or features may be provided. Aspects or features from one type (e.g., method or system) may be used in another type (system or method).

Illustrative Embodiment 1. A method for pose estimation of an ultrasound imaging catheter, the method comprising: imaging, by an ultrasound system, an internal region of a patient with the ultrasound imaging catheter, the imaging providing an image of the internal region; estimating a pose of the ultrasound imaging catheter, the pose estimated by a machine-learned model in response to input of the image to the machine-learned model; and displaying an indication of the pose.

Illustrative Embodiment 2. The method of Illustrative Embodiment 1, wherein the machine-learned model estimates the pose free of input from a position sensor.

Illustrative Embodiment 3. The method of any of Illustrative Embodiments 1-2, wherein the machine-learned model estimates the pose in response to input of only the image.

Illustrative Embodiment 4. The method of any of Illustrative Embodiments 1-3, wherein estimating the pose comprises estimating a position and an orientation of a transducer of the ultrasound imaging catheter, the pose comprising the position and the orientation in six degrees of freedom.

Illustrative Embodiment 5. The method of any of Illustrative Embodiments 1-4, wherein estimating the pose comprises estimating the pose relative to a center of an atrium.

Illustrative Embodiment 6. The method of any of Illustrative Embodiments 1-5, wherein estimating comprises estimating by the machine-learned model comprising a vision transformer.

Illustrative Embodiment 7. The method of Illustrative Embodiment 6, wherein estimating comprises dividing the image into patches with positional encoding and inputting the patches and positional encoding to the vision transformer, outputting a class token for each of the patches, and estimating the pose from the class tokens of the patches.

Illustrative Embodiment 8. The method of Illustrative Embodiment 7, wherein estimating comprises estimating the pose as a position and an orientation, the class tokens provided to a first linear layer for generating the position, and the class tokens provided to a second linear layer for generating the orientation.

Illustrative Embodiment 9. The method of any of Illustrative Embodiments 1-8, wherein displaying comprises displaying the indication as a graphic showing the pose of the ultrasound imaging catheter relative to anatomy of the patient.

Illustrative Embodiment 10. An ultrasound system for pose estimation, the ultrasound system comprising: an intracardiac echocardiogram (ICE) catheter with a transducer configured for ultrasound scanning from within a cardiac system of a patient; an image processor configured to determine a position and orientation of the ICE catheter by input of a scan from the ICE catheter to a neural network, the neural network configured by training to output the position and orientation in response to input of the scan; and a display configured to display the position and orientation.

Illustrative Embodiment 11. The ultrasound system of Illustrative Embodiment 10, wherein the neural network is configured to output the position and orientation in response to the input of the scan free of input of or derived from a position sensor.

Illustrative Embodiment 12. The ultrasound system of any of Illustrative Embodiments 10-11, wherein the image processor is configured to determine the position and orientation relative to anatomy of the patient.

Illustrative Embodiment 13. The ultrasound system of any of Illustrative Embodiments 10-12, wherein the neural network comprises a vision transformer.

Illustrative Embodiment 14. The ultrasound system of Illustrative Embodiment 13, wherein the scan comprises a spatial representation of the patient, and wherein the vision transformer is configured to operate on patches of the spatial representation with first and second linear output layers configured to output the position and orientation, respectively, in response to class tokens for the patches.

Illustrative Embodiment 15. The ultrasound system of any of Illustrative Embodiments 10-14, wherein the display of the position and orientation comprises a graphic showing the position and orientation relative to anatomy of the patient.

Illustrative Embodiment 16. A system for pose estimation of an imaging catheter, the system comprising: an image processor configured to generate a pose of the imaging catheter, the pose generated by a neural network configured by training to output the pose in response to input of an image from the imaging catheter to the neural network; and a display configured to display the pose.

Illustrative Embodiment 17. The system of Illustrative Embodiment 16, wherein the imaging catheter comprises an intracardiac echocardiogram (ICE) catheter and the pose is of a transducer of the ICE catheter.

Illustrative Embodiment 18. The system of any of Illustrative Embodiments 16-17, wherein the neural network is configured to output in response to the input of only the image.

Illustrative Embodiment 19. The system of any of Illustrative Embodiments 16-18, wherein the neural network comprises a vision transformer.

Illustrative Embodiment 20. The system of Illustrative Embodiment 19, wherein the vision transformer is configured to operate on patches of the image with first and second linear output layers configured to output the position and orientation, respectively, as the pose and in response to class tokens for the patches.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the scope of this invention.

**Claims**

1. A method for pose estimation of an ultrasound imaging catheter, the method comprising:

    imaging, by an ultrasound system, an internal region of a patient with the ultrasound imaging catheter, the imaging providing an image of the internal region;
    estimating a pose of the ultrasound imaging catheter, the pose estimated by a machine-learned model in response to input of the image to the machine-learned model; and
    displaying an indication of the pose.

2. The method of claim 1,
    wherein the machine-learned model estimates the pose free of input from a position sensor.

3. The method of claim 1,
    wherein the machine-learned model estimates the pose in response to input of only the image.

4. The method of any one of claims 1 - 3, wherein estimating the pose comprises:

   estimating a position and an orientation of a transducer of the ultrasound imaging catheter, the pose comprising the position and the orientation in six degrees of freedom;
   estimating the pose relative to a center of an atrium;
   estimating by the machine-learned model comprising a vision transformer; and/or
   dividing the image into patches with positional encoding and inputting the patches and positional encoding to the vision transformer, outputting a class token for each of the patches, and estimating the pose from the class tokens of the patches,
   wherein, in particular, the pose is estimated as a position and an orientation, the class tokens are provided to a first linear layer for generating the position, and the class tokens are provided to a second linear layer for generating the orientation.

5. The method of any one of claims 1 - 4, wherein displaying comprises displaying the indication as a graphic showing the pose of the ultrasound imaging catheter relative to anatomy of the patient.

6. An ultrasound system, in particular, for pose estimation, the ultrasound system comprising:

   an intracardiac echocardiogram (ICE) catheter with a transducer configured for ultrasound scanning from within a cardiac system of a patient;
   an image processor configured to determine a position and orientation of the ICE catheter by input of a scan from the ICE catheter to a neural network, the neural network configured by training to output the position and orientation in response to input of the scan; and
   a display configured to display the position and orientation.

7. The ultrasound system of claim 6, wherein the neural network is configured to output the position and orientation in response to the input of the scan free of input of or derived from a position sensor.

8. The ultrasound system of claim 6 or 7, wherein the image processor is configured to determine the position and orientation relative to anatomy of the patient.

9. The ultrasound system of claim 6 - 8, wherein the scan comprises a spatial representation of the patient, and wherein a vision transformer is configured to operate on patches of the spatial representation with first and second linear output layers configured to output the position and orientation, respectively, in response to class tokens for the patches.

10. The ultrasound system of any one of claims 6 - 9, wherein the display of the position and orientation comprises a graphic showing the position and orientation relative to anatomy of the patient.

11. A system for pose estimation of an imaging catheter, the system comprising:

    an image processor configured to generate a pose of the imaging catheter, the pose generated by a neural network configured to output the pose in response to input of an image from the imaging catheter to the neural network; and
    a display configured to display the pose.

12. The system of claim 11, wherein the imaging catheter comprises an intracardiac echocardiogram (ICE) catheter and the pose is of a transducer of the ICE catheter.

13. The system of any one of claims 6 - 12, wherein the neural network is configured to output in response to the input of only the image or only the scan.

14. The system of any one of claims 6 - 13, wherein the neural network comprises a vision transformer, wherein the vision transformer is, in particular, configured to operate on patches of the image with first and second linear output layers configured to output the position and orientation, respectively, as the pose and in response to class tokens for the patches.

15. The system of any one of claims 6 - 14, wherein the image processor is configured to carry out the step of estimating the pose of the method of any one of claims 1 - 5, the pose is a position and orientation of the catheter, and the machine-learned model is a neural network.

```
            ┌─────────────────────────────────────────────────┐
  100       │  Insert Probe into Patient                       │
            └─────────────────────────────────────────────────┘
                                  │
                                  ▼
            ┌─────────────────────────────────────────────────┐
  110       │  Image Patient                                   │
            └─────────────────────────────────────────────────┘
                                  │
                                  ▼
            ┌─────────────────────────────────────────────────┐
  120       │  Estimate Pose with ML Model from Imaging        │
            │  ┌───────────────────────────────────────────┐   │
  122       │  │  Divide Input into Patches                │   │
            │  ├───────────────────────────────────────────┤   │
  124       │  │  Input Patches to ML Model                │   │
            │  ├───────────────────────────────────────────┤   │
  126       │  │  Output Class Tokens                      │   │
            │  └───────────────────────────────────────────┘   │
            └─────────────────────────────────────────────────┘
                                  │
                                  ▼
            ┌─────────────────────────────────────────────────┐
  130       │  Displaying Indication of Pose                   │
            └─────────────────────────────────────────────────┘
```

FIG. 1

Input
(Current ICE image)

200

210

220
Pos Estimation Network

Vision
Transformer
222

224

Output

226

$[p_x, p_y, p_z]$

228

$[o_x, o_y, o_z]$

FIG. 2

FIG. 3

FIG. 4

FIG. 5

Europäisches
Patentamt
European
Patent Office
Office européen
des brevets

# EUROPEAN SEARCH REPORT

**Application Number**

EP 26 15 5825

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/301624 A1 (SALGAONKAR VASANT [US]) 28 September 2023 (2023-09-28) | 1-3,5-8, 10-13,15 | INV. A61B8/08 |
| Y | * paragraphs [0019] - [0045]; claims 1-20; figures 1-5 * | 4,9,14 | A61B8/12 A61B5/00 A61B8/00 |
| X | ARMIN MOHAMMAD ALI ET AL: "Learning Camera Pose from Optical Colonoscopy Frames Through Deep Convolutional Neural Network (CNN)", 8 September 2017 (2017-09-08), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER] SPRINGER, BERLIN, HEIDELBERG, PAGE(S) 50 - 59, XP047435531, ISSN: 0302-9743 ISBN: 9783540745495 [retrieved on 2017-09-08] * the whole document * | 11,13,15 | G06T7/00 |
| Y | HUH JAEYOUNG ET AL: "AI-Driven View Guidance System in Intra-Cardiac Echocardiography Imaging", IEEE TRANSACTIONS ON BIOMEDICAL ENGINEERING IEEE, USA, vol. 72, no. 7, 24 January 2025 (2025-01-24), pages 2072-2084, XP034918728, ISSN: 0018-9294, DOI: 10.1109/TBME.2025.3533485 [retrieved on 2025-01-24] * the whole document * | 4,9,14 | TECHNICAL FIELDS SEARCHED (IPC) A61B G06T |

The present search report has been drawn up for all claims

| Place of search | Date of completion of the search | Examiner |
|---|---|---|
| Munich | 18 June 2026 | Daoukou, Eleni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 26 15 5825

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2026

| Patent document cited in search report | Publication date | Patent family member(s) | Publication date |
|---|---|---|---|
| US 2023301624 A1 | 28-09-2023 | CN 113661544 A | 16-11-2021 |
| | | DE 112020001809 T5 | 03-02-2022 |
| | | KR 20210149112 A | 08-12-2021 |
| | | KR 20240055167 A | 26-04-2024 |
| | | US 2020315572 A1 | 08-10-2020 |
| | | US 2023301624 A1 | 28-09-2023 |
| | | WO 2020205187 A1 | 08-10-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63753522